# EUROPÄISCHE PATENTANMELDUNG

(11) **EP 0 711 750 A1**
(43) Veröffentlichungstag der Anmeldung: **15.05.1996**
(21) Anmeldenummer: 95116938.2
(22) Anmeldetag: 27.10.1995
(51) Int. Cl.: C07C 209/62, C07C 211/37

(54) **Verfahren zur Herstellung von 2-Fluorcyclopropylamin und neue Cyclopropylaminderivate**

(30) Priorität: 09.11.1994 DE 4440021
(71) Anmelder: BAYER AG, D-51368 Leverkusen (DE)
(72) Erfinder: Lui, Norbert, Dr., D-51061 Köln (DE); Marhold, Albrecht, Dr., D-51373 Leverkusen (DE); Böhm, Stefan, Dr., D-47800 Krefeld (DE)

(57) **Zusammenfassung**

2-Fluor-cyclopropylamin wird in vorteilhafter Weise hergestellt, indem man eine N-Vinylverbindung mit einem Carben des Typs FXC: (mit X = Chlor oder Brom) umsetzt, so das entsprechende Cyclopropylaminderivat erhält, in diesem den Rest X durch Reduktion gegen Wasserstoff austauscht und abschließend die sonstigen Substituenten am Stickstoffatom abspaltet.

## Beschreibung

Die vorliegende Erfindung betrifft ein besonders vorteilhaftes Verfahren zur Herstellung von 2-Fluorcyclopropylamin aus N-Vinylverbindungen und neue Cyclopropylaminderivate, die bei diesem Verfahren als Zwischenprodukte anfallen.

Es sind bereits zwei Wege zur Herstellung von 2-Fluorcyclopropylamin bekannt. Bei einem Weg geht man von Butadien aus, das man mit einem Fluor-Halogen-Carben in ein Vinylhalogenfluorcyclopropanderivat überführt, und aus diesem über die Stufen: Oxidation zur Carbonsäure, reduktive Entfernung des Halogenatoms, Überführung in das Säurechlorid, Umsetzung mit einem Azid zum entsprechenden Isocyanat und dessen Verseifung, das 2-Fluorcyclopropylamin gewinnt (s. J. Fluorine Chem. 49, 127-139 (1990)). Neben der Vielstufigkeit dieses Verfahrens ist die schwierige Zugänglichkeit und Handhabbarkeit des benötigten Azids und die geringe Ausbeute bei einigen Teilschritten nachteilig. So wird bei der Umsetzung mit dem Carben nur eine Ausbeute von 31 % und bei der reduktiven Entfernung des Halogenatoms nur eine Ausbeute von 10 % erzielt. Im Gesamtverfahren fällt 2-Fluorcyclopropylamin in einer Ausbeute von weniger als 3 % an.

Bei dem zweiten Weg setzt man N-Benzyl-N-vinyl-carbamat mit dem Carben HFC: um und spaltet aus dem Reaktionsprodukt die Benzyl- und die Carbamatgruppe ab (s. Tetrahedron 50, (13) 3889-3904 (1994)). CHFJ₂, das Ausgangsprodukt für das Carben HFC:, muß in mehreren Stufen aus diversen Reagenzien (Kaliumiodid, Antimon(III)-fluorid, Tribrommethan) hergestellt und zur Überführung in HFC: mit dem selbstentzündlichen Diethylzink in Kontakt gebracht werden. Dieses Verfahren ist zu umständlich und zu teuer, um im technischen Maßstab angewendet zu werden.

Es besteht also immer noch das Bedürfnis nach einem weniger aufwendigen Verfahren zur Herstellung von 2-Fluorcyclopropylamin, das auch in technischem Maßstab durchführbar ist und gute Ausbeuten erbringt.

Es wurde nun ein Verfahren zur Herstellung von 2-Fluorcyclopropylamin gefunden, das dadurch gekennzeichnet ist, daß man eine N-Vinylverbindung der Formel 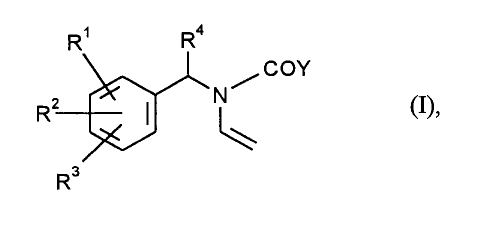
in der
- R¹, R² und R³: unabhängig voneinander jeweils für Wasserstoff, C₁-C₆-Alkyl, Halogen, C₁-C₆-Halogenalkyl oder C₁-C₆-Alkoxy,
- R⁴: für Wasserstoff C₁-C₆-Alkyl oder C₆-C₁₂-Aryl und
- Y: für offenkettiges oder cyclisches C₁-C₁₂-Alkoxy, C₆-C₁₂-Aryl, C₇-C₁₂-Aralkyl, C₇-C₁₂-Aralkyloxy oder offenkettiges oder cyclisches C₁-C₁₂-Alkyl stehen,
mit einem Carben des Typs FXC:, mit X = Chlor oder Brom, umsetzt, so ein Cyclopropylaminderivat der Formel (II) erhält 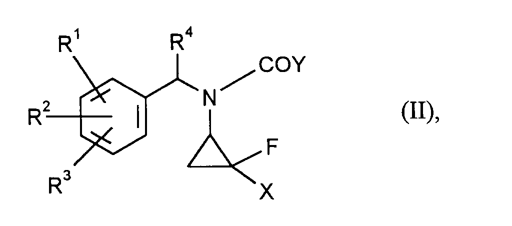
in der
R¹, R², R³, R⁴, X und Y die oben angegebene Bedeutung haben, aus dem Cyclopropylaminderivat der Formel (II) durch Reduktion X gegen Wasserstoff austauscht und abschließend die COY- und die, gegebenenfalls substituierte, Benzylgruppe vom Stickstoffatom abspaltet.

Soweit R¹, R² und R³ Halogen oder C₁-C₆-Halogenalkyl bedeuten, kann es sich bei Halogen z.B. um Fluor, Chlor oder Brom und bei C₁-C₆-Halogenalkyl z.B. um 1 bis 3 Fluor-, Chlor- und/oder Bromatome enthaltendes C₁-C₆-Halogenalkyl handeln.

R¹ steht vorzugsweise für Wasserstoff, Methyl, Ethyl, Chlor, Methoxy oder Ethoxy. R² und R³ stehen vorzugsweise für Wasserstoff. R⁴ steht vorzugsweise für Wasserstoff, Methyl, Ethyl oder Phenyl. Y steht vorzugsweise für Methyl, Ethyl, Phenyl, t-Butyloxy, Benzyloxy oder Menthyloxy. X steht vorzugsweise für Chlor.

Die Herstellung von Verbindungen der Formel (I) ist literaturbekannt (siehe z.B. Tetrahedron 50, 3889 (1994)) oder kann analog dazu erfolgen.

Carbene des Typs FXC: können z.B. in situ aus Dichlor- oder Dibrom-fluormethan und Alkyllithium bei tiefen Temperaturen (siehe Chem. Ber. 104, 1921 (1971)), aus Dichlor- oder Dibrom-fluormethanen und einem Oxiran in Gegenwart von Tetraalkyl-, -aryl- oder -aralkyl-ammonium-bromid (siehe Chem. Ber. 100, 1858 (1967)), aus Dichlor- oder Dibrom-fluormethan mit Natronlauge in Gegenwart eines Phasentransferkatalysators (siehe J. Fluorine Chem. 49, 127 (1990)) oder durch Umsetzung von Natriummethylat mit Dichlorfluoressigsäuremethylester (siehe Tetrahedron Letters 1967, 1123) hergestellt werden.

Vorzugsweise verfährt man so, daß man in ein Gemisch enthaltend eine Verbindung der Formel (I), wäßrige Natronlauge, ein inertes, mit Wasser nicht mischbares Lösungsmittel und einen Phasentransferkatalysator bei -20 bis +50°C, vorzugsweise -10 bis +30°C Dichlorfluormethan einleitet oder bei 0 bis 40°C Dibromfluormethan zutropft.

Die wäßrige Natronlauge kann z.B. eine Konzentration von 30 bis 55 Gew.-%, vorzugsweise von 45 bis 50 Gew.-% aufweisen. Bezogen auf 100 g der Verbindung der Formel (I) kann man z.B. 50 bis 1 000 ml wäßrige Natronlauge einsetzen. Vorzugsweise liegt diese Menge bei 80 bis 600 ml.

Als inertes, mit Wasser nicht mischbares Lösungsmittel, kommt z.B. Methylenchlorid, Toluol, Chlorbenzol oder Trichlortrifluorethan in Frage. Bevorzugt ist Dichlormethan. Bezogen auf 100 g der Verbindung der Formel (I) kann man z.B. 40 bis 800 ml des Lösungsmittels einsetzen. Vorzugsweise beträgt diese Menge 70 bis 700 ml.

Dichlorfluormethan (R21) ist ein im Handel erhältliches technisches Gas. Dibromfluormethan ist z.B. gemäß Chem. Ber. 104, 1934-1941 (1971) zugänglich. Bezogen auf 1 Mol der Verbindung der Formel (I) kann man z.B. 1 bis 10 Mole Dichlor- oder Dibrom-fluormethan einsetzen. Vorzugsweise liegt diese Menge bei 1 bis 5 Molen.

Als Phasentransferkatalysatoren kommen z.B. quarternäre Ammoniumsalze wie Benzyltriethylammoniumchlorid und Tetrabutylammoniumbromid oder Kronenether wie 18-Krone-6 und Dicyclohexano-18-Krone-6 oder quarternäre Phosphoniumsalze in Frage. Phasentransferkatalysatoren kann man z.B. in Mengen von 0,1 bis 10, vorzugsweise 0,5 bis 8 Mol.-%, bezogen auf die Verbindung der Formel (I), einsetzen.

Der Druck bei der Umsetzung mit dem vorzugsweise in situ gebildeten Carben ist nicht kritisch. Er kann beispielsweise im Bereich 0,1 bis 40 bar liegen. Bevorzugt sind Normaldruck und Drucke von 1 bis 6 bar.

Die Aufarbeitung des nach der Umsetzung mit dem Carben vorliegenden Gemisches kann z.B. erfolgen, indem man das Gemisch mit Wasser oder Eis verdünnt, mit einem inerten, mit Wasser nicht mischbaren Lösungsmittel, vorzugsweise Dichlormethan, extrahiert, das Extrakt trocknet und abschließend das Lösungsmittel, vorzugsweise im Vakuum, entfernt. Man erhält so das der eingesetzten Verbindung der Formel (I) und dem verwendeten Carben entsprechende Cyclopropylaminderivat der Formel (II).

Das Cyclopropylaminderivat der Formel (II) wird nunmehr einer Reduktion unterworfen, bei der der Substituent X gegen Wasserstoff ausgetauscht wird. Die Reduktion kann z.B. mit Tributylzinnhydrid in Gegenwart eines Radikalstarters, z.B. Azoisobuttersäurenitril, erfolgen. Weitere mögliche Reduktionsmittel sind Natriumborhydrid/Tributylzinnchlorid, Organosiliconhydride in Gegenwart eines Radikalstarters und Calcium/Ammoniak. Man kann die Reduktion auch auf elektrochemischen Weg durchführen sowie mit Wasserstoff in Gegenwart von Raney-Nickel, gegebenenfalls in Gegenwart einer Base. Cyclopropylaminderivate der Formel (II) mit X = Brom können auch mit Zink reduziert werden.

Es ist vorteilhaft, das Reduktionsmittel im Überschuß einzusetzen. Die Temperatur bei der Reduktion kann je nach verwendetem Reduktionsmittel unterschiedlich gewählt werden. Sie darf nicht so hoch sein, daß der aromatische oder der Cyclopropyl-Ring der Verbindung der Formel (II) angegriffen wird. Man bleibt deshalb im allgemeinen unter 170°C, vorzugweise unter 150°C. Beim Einsatz von Hydriden sind Temperaturen im Bereich 70 bis 120°C bevorzugt, beim Einsatz von Zink solche im Bereich 10 bis 60°C, bei elektrochemischer Reduktion solche im Bereich 0 bis 50°C und beim Einsatz von Calcium/Ammoniak solche im Bereich -40 bis -20°C. Besonders bevorzugt arbeitet man mit Tributylzinnhydrid und Azoisobuttersäurenitril und bei 70 bis 100°C. Zweckmäßigerweise entfernt man überschüssiges Reduktionsmittel nach Beendigung der Reduktion, z.B. durch Destillation beim Einsatz von Hydriden oder durch wäßrige Aufarbeitung und Extraktion des Reaktionsprodukts mit einem organischen Lösungsmittel beim Einsatz von Calcium/Ammoniak oder Zink.

Die Abspaltung der jetzt noch am Stickstoffatom befindlichen COY-Gruppe und der gegebenenfalls substituierten Benzylgruppe kann auf an sich bekannte Weise erfolgen, z.B. nach Methoden, wie sie in Tetrahedron 50, 3889-3904 (1994) und in der JP-OS 06-092 911 beschrieben sind.

Das erfindungsgemäße Verfahren gestattet die Herstellung von 2-Fluorcyclopropylamin in guten Ausbeuten, ohne schwierig handzuhabende und ohne nur sehr aufwendig zugängliche Hilfsmittel. Außerdem ist die technische Durchführung wesentlich einfacher als bei bisher bekannten Verfahren. Das Auffinden des erfindungsgemäßen Verfahrens war besonders überraschend, denn beim Einsatz von N-Vinylphthalimid anstelle einer Verbindung der Formel (I) und sonst gleichen Reaktionsbedingungen fand keine Umsetzung statt (siehe Beispiel 1). Außerdem findet beim Einsatz optisch aktiver Verbindungen der Formel (I), das sind solche mit R⁴ ≠ Wasserstoff, keine Racemisierung statt.

Die Cyclopropylaminderivate der Formel (II), die ein Schlüsselprodukt im erfindungsgemäßen Verfahren zur Herstellung von 2-Fluorcyclopropylamin sind, sind neu. Die vorliegende Erfindung betrifft deshalb auch neue Cyclopropylaminderivate der Formel 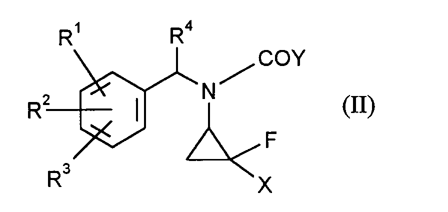
in der
- R¹, R² und R³: unabhängig voneinander jeweils für Wasserstoff, C₁-C₆-Alkyl, Halogen, C₁-C₆-Halogenalkyl oder C₁-C₆-Alkoxy,
- R⁴: für Wasserstoff, C₁-C₆-Alkyl oder C₆-C₁₂-Aryl, und
- Y: für offenkettiges oder cyclisches C₁-C₁₂-Alkoxy, C₆-C₁₂-Aryl, C₇-C₁₂-Aralkyl, C₇-C₁₂-Aralkyloxy oder offenkettiges oder cyclisches C₁-C₁₂-Alkyl und
- X: für Chlor oder Brom stehen.

Bevorzugte Cyclopropylaminderivate der Formel (II) sind solche, bei denen
- R¹: für Wasserstoff, Methyl, Ethyl, Chlor, Methoxy oder Ethoxy,
- R² und R³: für Wasserstoff,
- R⁴: für Wasserstoff, Methyl, Ethyl oder Phenyl,
- Y: für Methyl, Ethyl, Phenyl, t-Butoxy, Benzyloxy oder Menthyloxy und
- X: für Chlor stehen.

Besonders bevorzugte Cyclopropylaminderivate der Formel (II) sind N-Benzyl-N-(2-chlor-2-fluorcyclopropyl)-benzylcarbamat, N-Benzyl-N-(2-brom-2-fluorcyclopropyl)-carbamat, N-Benzyl-N-cis und trans(2-chlor-2-fluorcyclopropyl)-acetamid, N-(R)-α-Methylbenzyl-N-(2-chlor-2-fluorcyclopropyl)-benzylcarbamat, N-(R)-α-Methylbenzyl-N-(2-brom-2-fluorcyclopropyl)-benzylcarbamat und N-Benzyl-N-(2-brom-2-fluorcyclopropyl)-(1R, 2S, 5R)-p-menthylcarbamat.

Ein Verfahren zur Herstellung von Cyclopropylaminderivaten der Formel (II) und deren Verwendung zur vorteilhaften Herstellung von 2-Fluorcyclopropylamin ist weiter oben beschrieben.

### Beispiele

### Beispiel 1 ( zum Vergleich)

Zu 30 g 45 gew.%ige Natronlauge wurden bei 0°C 18 g N-Vinylphthalimid gelöst in 30 ml Methylenchlorid zugetropft. Nach Zugabe von 1 g Benzyltriethylammoniumchlorid wurden bei 0 bis 5°C 40 g Dichlorfluormethan langsam eingeleitet. Nach Beendigung der Reaktion wurde die Mischung auf Eis gegossen, die organische Phase abgetrennt und die wäßrige Phase mit Methylenchlorid extrahiert. Nach Trocknung der vereinigten organischen Phasen und Entfernung des Lösungsmittels wurde nach einer GC-MS-spektroskopischen Untersuchung keine Umsetzungsprodukt des N-Vinylphthalimids gefunden.

### Beispiel 2

Zu einer Mischung aus 50 g N-Benzyl-N-vinylbenzylcarbamat, 80 ml 45 %iger wäßrige Natronlauge, 50 ml Methylenchlorid und 0,5 g Benzyltriethylammoniumchlorid wurden innerhalb von 2 h bei 5 bis 10°C 41 g Dichlorfluormethan zudosiert. Die Mischung wurde anschließend mit Wasser verdünnt und mit Methylenchlorid extrahiert. Nach Trocknung über Natriumsulfat wurde aus dem Extrakt das Lösungsmittel im Vakuum entfernt. Der Umsatz war gemäß GC-MS vollständig, und es wurden 37 g N-Benzyl-N-(2-chlor-2-fluorcyclopropyl)-benzylcarbamat erhalten (Ausbeute= 59 % d Th.).

Das ¹H-NMR-Spektrum in Deuterochloroform zeigte charakteristische Resonanzen bei δ: 1,3-1,9 (m, 2H), 2,9 und 3,15 (m, 1H), 4,2-4,9 (m, 2H), 5,22 (s, 2H) und 7,1-7,5 (m, 10H) ppm.

Das ¹⁹F-NMR Spektrum in Deuterochloroform zeigte charakteristische Resonanzen bei δ: -137 und -152 ppm.

### Beispiel 3

Zu einer Mischung aus 24 g N-Benzyl-N-vinylbenzylcarbamat, 70 ml 45 gew.-%iger wäßriger Natronlauge, 60 ml Methylenchlorid und 1 g Benzyltriethylammoniumchlorid wurden innerhalb von 1,5 Stunden bei 5 bis 10°C 21 g Dibromfluormethan zugetropft. Anschließend wurde die Mischung mit Wasser verdünnt und mit Methylenchlorid extrahiert. Nach Trocknung des Extraktes über Natriumsulfat wurde das Lösungsmittel im Vakuum entfernt. Es wurden 25,8 g N-Benzyl-N-(2-brom-2-fluorcyclopropyl)-benzylcarbamat isoliert (Ausbeute: 75 % d.Th.).

Das ¹H-NMR-Spektrum in Deuterochloroform zeigte charakteristische Resonanzen bei δ: 1,4-1,95 (m, 2H), 2,9-3,15 (m, 1H), 4,2-4,9 (m, 2H), 5,2 (s, 2H) und 7,1-7,5 (m, 10H) ppm.

Das ¹⁹F-NMR-Spektrum in Deuterochloroform zeigte charakteristische Resonanzen bei δ: -134,5 und bei -150,5 ppm.

### Beispiel 4

Zu einer Mischung aus 100 g N-Benzyl-N-vinylacetamid, 400 ml 45 gew.-%iger wäßriger Natronlauge, 400 ml Methylenchlorid und 4 g Benzyltriethylammoniumchlorid wurden innerhalb von 2 Stunden 83 g Dichlorfluormethan bei 5 bis 10°C zudosiert. Die Mischung wurde anschließend mit Wasser verdünnt und mit Methylenchlorid extrahiert. Nach der Trocknung des Extraktes über Natriumsulfat wurde das Lösungsmittel im Vakuum entfernt. Es hinterblieb nach einem Umsatz von 50 % (bestimmt per GC) ein cis-trans-Isomerengemisch (Verhältnis 1:2) von N-Benzyl-N-cis-(2-chlor-2-fluorcyclopropyl)-acetamid und N-Benzyl-N-trans-(2-chlor-2-fluorcyclopropyl)-acetamid.

Das ¹⁹F-NMR-Spektrum in Deuterochloroform zeigte charakteristische Resonanzen bei δ = -136,4 und -149,7 ppm.

### Beispiel 5

Zu einer Mischung von 95 g N-(R)-α-Methylbenzyl-N-vinylbenzylcarbamat, 100 ml 45 gew.-%iger wäßriger Natronlauge, 100 ml Methylenchlorid und 2 g Benzyltriethylammoniumchlorid wurden innerhalb von 3 Stunden bei 5 bis 10°C 95 g Dichlorfluormethan eingeleitet. Anschließend wurde die Mischung mit Wasser verdünnt und mit Methylenchlorid extrahiert. Nach Trocknung des Extraktes über Natriumsulfat wurde das Lösungsmittel im Vakuum entfernt. Ausbeute 54 % (bestimmt durch NMR) N-(R)-α-Methylbenzyl-N-(2-chlor-2-fluorcyclopropyl)-benzylcarbamat.

Das ¹H-NMR-Spektrum in Deuterochloroform zeigte charakteristische Resonanzen bei δ: 1,3-1,7 (m, 2H), 1,7 (t, 3H), 2,75-3,3 (m, 1H), 5,1-5,5 (m, 3H) und 7,2-7,5 (m, 10H) ppm.

Das ¹⁹F-NMR Spektrum in Deuterochloroform zeigte charakteristische Resonanzen bei δ: -138,3 und bei -150,6 ppm.

### Beispiel 6

Zu einer Mischung von 56 g N-(R)-α-Methylbenzyl-N-vinylbenzylcarbamat, 55 ml 45 gew.-%iger wässriger Natronlauge, 60 ml Methylenchlorid und 1 g Benzyltriethylammoniumchlorid wurde innerhalb von 2 Stunden bei 5 bis 10°C 48 g Dibromfluormethan zugetropft. Anschließend wurde die Mischung mit Wasser verdünnt und mit Methylenchlorid extrahiert. Nach Trocknung des Extraktes über Natriumsulfat wurde das Lösungsmittel im Vakuum entfernt. Ausbeute 57 % (bestimmt durch NMR) N-(R)-α-Methylbenzyl-N-(2-brom-2-fluorcyclopropyl)-benzylcarbamat.

Das ¹H-NMR Spektrum in Deuterochloroform zeigte charakteristische Resonanzen bei δ: 1,4-1,95 (m, 2H), 1,76 (t, 3H), 2,9-3,2 (m, 1H), 5,1-5,5 (m, 3H) und 7,2-7,5 (m, 10H) ppm.

Das ¹⁹F-NMR Spektrum in Deuterochloroform zeigte charakteristische Resonanzen bei δ: -136 und bei -149,9 ppm.

### Beispiel 7

Zu einer Mischung von 50 g N-Benzyl-N-vinyl-(1R, 2S, 5R)-p-menthyl-carbamat, 80 ml 45 gew.-%iger wässriger Natronlauge, 100 ml Methylenchlorid und 2 g Benzyltriethylammoniumchlorid wurden innerhalb von 2 Stunden bei 5 bis 10°C 81 g Dichlorfluormethan eingeleitet. Anschließend wurde die Mischung mit Wasser verdünnt und mit Methylenchlorid extrahiert. Nach Trocknung des Extraktes über Natriumsulfat wurde das Lösungsmittel im Vakuum entfernt. Ausbeute: 70 % (bestimmt durch NMR) N-Benzyl-N-(2-brom-2-fluorcyclopropyl)-(1R, 2S, 5R)-p-menthyl-carbamat.

Das ¹H-NMR Spektrum in Deuterochloroform zeigte charakteristische Resonanzen bei δ: 0,7-2,2, 2,7-3,3, 4,25, 4,6-4,9 und 7,3 ppm.

Das ¹⁹F-NMR Spektrum in Deuterochloroform zeigte charakteristische Resonanzen bei δ: -137 und bei -152 ppm.

### Beispiel 8

10 g N-(R)-α-Methylbenzyl-N-(2-chlor-2-fluorcyclopropyl)-benzylcarbamat, 9,2 g Tributylzinnhydrid und 0,3 g Azoisobuttersäurenitril (AIBN) wurden auf 80-90°C erwärmt. Anschließend wurde 4 Stunden bei 80 bis 90°C gerührt und wieder 0,3 g AIBN zugefügt. Nach weiterem 4-stündigem Rühren wurde die Reaktionsmischung abgekühlt und auf Kieselgel säulenchromatographisch getrennt (n-Hexan/MTBE 5:1).
Ausbeute: 2,8 g (=32 %) trans-N-(R)-α-Methylbenzyl-N-(2-fluorcyclopropyl)-benzylcarbamat und 2,73 g (31 %) cis-N-(R)-α-Methylbenzyl-N-(2-fluorcyclopropyl)-benzylcarbamat, also insgesamt 63 %. Die physikalischen Daten stimmten mit den Literaturwerten überein.

### Beispiel 9

Die Produkte des Beispiels 8 wurden getrennt einer Hydrierung zur Abspaltung der Benzylgruppe und einer sauren Abspaltung der Carbamatgruppe in der in Tetrahedron 50, 3889-3904 (1994) beschriebenen Weise unterworfen und so 2-Fluorcyclopropylamin in beiden Fällen in den dort angegebenen Ausbeuten erhalten.

## Patentansprüche

1. Verfahren zur Herstellung von 2-Fluor-cyclopropylamin, dadurch gekennzeichnet, daß man eine N-Vinylverbindung der Formel 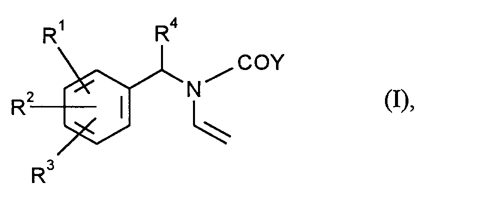 in der
R¹, R² und R³ unabhängig voneinander jeweils für Wasserstoff, C₁-C₆-Alkyl, Halogen, C₁-C₆-Halogenalkyl oder C₁-C₆-Alkoxy,
R⁴ für Wasserstoff, C₁-C₆-Alkyl oder C₆-C₁₂-Aryl und
Y für offenkettiges oder cyclisches C₁-C₁₂-Alkoxy, C₁-C₁₂-Aryl, C₇-C₁₂-Aralkyl, C₇-C₁₂-Aralkyloxy oder C₁-C₁₂-Alkyl stehen,
mit einem Carben des Typs FXC:, mit X = Chlor oder Brom, umsetzt, so ein Cyclopropylaminderivat der Formel (II) erhält 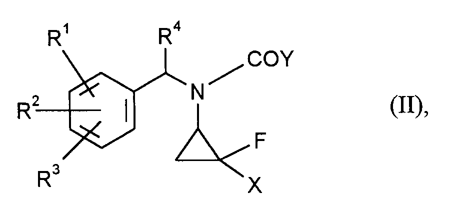 in der
R¹, R², R³, R⁴, X und Y die oben angegebene Bedeutung haben,
aus dem Cyclopropylaminderivat der Formel (II) durch Reduktion X gegen Wasserstoff austauscht und abschließend die COY- und die, gegebenenfalls substituierte, Benzylgruppe vom Stickstoffatom abspaltet.

2. Verfahren nach Anspruch 1, dadurch gekennzeichnet, daß in den Formeln (I) und (II)
R¹ für Wasserstoff, Methyl, Ethyl, Chlor, Methoxy oder Ethoxy,
R² und R³ für Wasserstoff,
R⁴ für Wasserstoff, Methyl, Ethyl oder Phenyl und
Y für Methyl, Ethyl, Phenyl, t.-Butyloxy, Benzyloxy oder Menthyloxy stehen und
in Formel (II) X für Chlor steht.

3. Verfahren nach Ansprüchen 1 und 2, dadurch gekennzeichnet, daß man die Umsetzung mit dem Carben so durchführt, daß man in ein Gemisch enthaltend eine Verbindung der Formel (I), wäßrige Natronlauge, ein inertes, mit Wasser nicht mischbares Lösungsmittel und einen Phasentransferkatalysator bei -20 bis +50°C Dichlorfluormethan einleitet oder bei 0 bis 40°C Dibromfluormethan zutropft.

4. Verfahren nach Ansprüchen 1 bis 3, dadurch gekennzeichnet, daß man bei dem Cyclopropylaminderivat der Formel (II) den Substituenten X gegen Wasserstoff austauscht, indem man mit Tributylzinnhydrid in Gegenwart eines Radikalstarters, Natriumborhydrid/Tributylzinnchlorid, Organosiliconhydriden in Gegenwart eines Radikalstarters oder Calcium/Ammoniak oder auf elektrochemischem Weg reduziert.

5. Verfahren nach Ansprüchen 1 bis 4, dadurch gekennzeichnet, daß man die nach dem Austausch des Substituenten X gegen Wasserstoff am Stickstoffatom noch vorhandene COY-Gruppe und die gegebenenfalls substituierte Benzylgruppe nach Methoden abspaltet wie sie in Tetrahedron 50, 3889-3904 (1994) und in der JP-OS 06-092 911 beschrieben sind.

6. Cyclopropylaminderivate der Formel 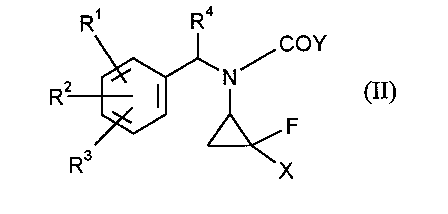 in der
R¹, R² und R³ unabhängig voneinander jeweils für Wasserstoff C₁-C₆-Alkyl, Halogen, C₁-C₆-Halogenalkyl oder C₁-C₆-Alkoxy,
R⁴ für Wasserstoff, C₁-C₆-Alkyl oder C₆-C₁₂-Aryl, und
Y für offenkettiges oder cyclisches C₁-C₁₂-Alkoxy, C₆-C₁₂-Aryl, C₇-C₁₂-Aralkyl, C₇-C₁₂-Aralkyloxy, oder offenkettiges oder cyclisches C₁-C₁₂-Alkyl und
X für Chlor oder Brom stehen.

7. Cyclopropylaminderivate nach Anspruch 6, dadurch gekennzeichnet, daß
R¹ für Wasserstoff Methyl, Ethyl, Chlor, Methoxy oder Ethoxy,
R² und R³ für Wasserstoff,
R⁴ für Wasserstoff, Methyl, Ethyl oder Phenyl,
Y für Methyl, Ethyl, Phenyl, t.-Butyloxy, Benzyloxy oder Menthyloxy und
X für Chlor stehen.

8. Cyclopropylaminderivate nach Ansprüchen 6 und 7, dadurch gekennzeichnet, daß es sich um N-Benzyl-N-(2-chlor-2-fluorcyclopropyl)-benzylcarbamat, N-Benzyl-N-(2-brom-2-fluorcyclopropyl)-carbamat, N-Benzyl-N-cis und trans(2-chlor-2-fluorcyclopropyl)-acetamid, N-(R)-α-Methylbenzyl-N-(2-chlor-2-fluorcyclopropyl)-benzylcarbamat, N-(R)-α-Methylbenzyl-N-(2-brom-2-fluorcyclopropyl)-benzylcarbamat und N-Benzyl-N-(2-brom-2-fluorcyclopropyl)-(1R, 2S, 5R)-p-menthylcarbamat.
